# EUROPEAN PATENT APPLICATION

(11) **EP 4 280 222 A1**
(43) Date of publication of application: **22.11.2023**
(21) Application number: 22174571.4
(22) Date of filing: 20.05.2022
(51) Int. Cl.: G16H 30/40

(54) **MEDICAL IMAGING WITH DISTRIBUTED CLUSTER-BASED PROCESSING**

(71) Applicant: Siemens Healthcare GmbH, 91052 Erlangen (DE)
(72) Inventor: CAMPAGNA, Swen, 91238 Engelthal (DE); STRANJAK, Armin, 91080 Uttenreuth (DE); HUANG, Yan Tu, 518000 Shenzhen (CN)

(57) **Abstract**

A local control device (21) for a medical imaging system (20) is described. The local control device (21) comprises a measurement control unit (4) for dividing a computing task (T) into a sequence (S) of consecutive sub-tasks (T₁, ..., Tₙ), wherein the computing task (T) is related to a generation of image data (BD) based on measurement data. The local control device (21) also includes an execution unit (24a) for executing the computing task (T). Further, the local control device (21) comprises a selection unit (24b) for selecting a sub-task (..., Tₙ₋₁, Tₙ) of the consecutive sub-tasks (T₁, ..., Tₙ) for external execution. Furthermore, the local control device (21) comprises an outsourcing unit (24c) for outsourcing the selected sub-task (..., Tₙ₋₁, Tₙ) to a remote computing source (RCSₖ, 21', 21", 26) for remotely generating a partial computing result (CRₖ) and for receiving the generated partial computing result (CRₖ). The execution unit (24a) is arranged to execute the computing task (T) based on the received generated partial computing result (CRₖ). Further, a medical imaging system (20) is described. Besides, a system-cluster (40) is described. Furthermore, a method for controlling a medical imaging system (20) is described.

## Description

The invention relates to a local control device for a medical imaging system. The invention also concerns a medical imaging system. Further, the invention relates to a system-cluster. Furthermore, the invention relates to method for controlling a medical imaging system.

Medical imaging processes (for example MR imaging processes) depict the inside of the body of a patient, either in full or in part. Imaging techniques help doctors to diagnose a disease, to assess its severity and to monitor sick patients. Most imaging procedures are painless, relatively safe, and non-invasive. Medical imaging includes, for example, imaging techniques based on radiation, ultrasound scans and magnetic resonance imaging (abbreviated as MRI or MR imaging).

Imaging modalities, in particular magnetic resonance imaging systems (abbreviated by MRI system or MR system), need substantial computing power for quickly performing preparation, execution and evaluation, in particular a reconstruction of images based on raw data, of an imaging process. It is important that reconstructed images are available to the operator of a scan unit shortly after executing the measurement, i.e. the acquisition of raw data. Short time of an image reconstruction is crucial, in particular in emergency circumstances where patient's health could be at risk.

For a magnetic resonance imaging system, two different approaches are conventionally used for achieving a sufficient speed of reconstruction. The first is to equip an MR system with sufficient local centralized computing power, basically related to the number of receive channels. Receive channels are used for receiving magnet resonance signals from an examination portion. However, that approach needs many resources. The second approach includes an offloading of the computing task, especially the image reconstruction, somewhere else, for example somewhere in the cloud, i.e. in a cloud computing system. Hence, the MR system itself requires less computing power, but it is dependent on an external computing resource, which has to be paid also. Further, either bandwidth limitations or poor network structure impede this approach significantly.

Conventionally, a computing task, for example an image reconstruction, is designed in a monolithic computing model as a default, i.e. the whole computation is run as a single complete computing task.

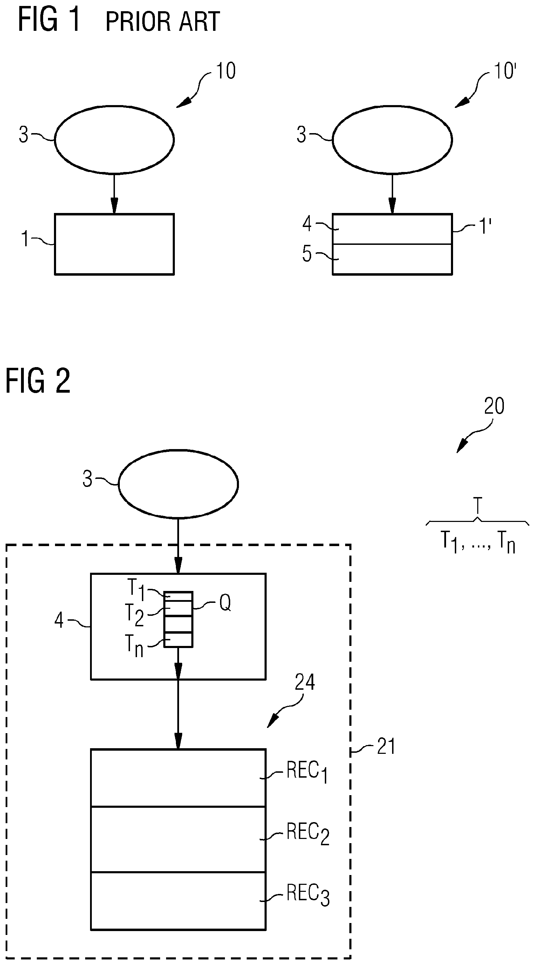

In FIG 1, an MR system with an associated computer, also named as measurement and reconstruction system (abbreviated by the acronym MARS), is depicted. Such measurement and reconstruction system can include two separate computers or one single computer. In both cases, the reconstruction is typically implemented monolithically, i.e. it is executed on one single computer.

It is desirable to have enough capacities for executing the necessary calculations for imaging independently, however to be capable also to use extern resources for accelerating the imaging process. Today, a computer of a medical imaging system may be able to communicate with external resources like a cloud computing system or other medical imaging systems. However, an external calculation of an important process makes the medical imaging system dependent on a present capacity of communication lines and on the capacity of the remote resources. However, reliability of schedule of a control and an imaging generating process is crucial for an efficient use of medical imaging capacities.

Hence, there is a problem of an increase of speed of medical imaging while ensuring the reliability of imaging operations.

The before-mentioned problem is solved by a local control device for a medical imaging system according to claim 1, a medical imaging system according to claim 10, a system-cluster according to claim 12 and a method for controlling a medical imaging system according to claim 13.

The local control device for a medical imaging system comprises a measurement control unit for dividing a computing task into a sequence of consecutive sub-tasks. As later described, the sub-tasks may be independent of each other, for example, they may include independent images. Thus, the ordering into a sequence is arbitrary in that case. As local control device, a control device which is positioned at the location of an assigned medical imaging system and which is intended to control the assigned medical imaging system is to be understood. The computing task is related to the generation of image data based on measurement data and preferably to a control task for controlling an imaging process or a task for processing measurement data for generating image data. In particular, the computing task is related to the generation of control data to be transmitted to a scan unit and/or to the processing of raw data received from the scan unit. As later discussed in detail, the computing task related to the process of generating image data preferably includes a reconstruction task or a filtering task.

In particular a complicated reconstruction task, for example a parallel reconstruction, may divided into sub-tasks.

A parallel reconstruction may be applied to an acquisition of different images in parallel, for example by interleaving k-space lines of different images. A parallel reconstruction can be also applied to a computationally parallel image reconstruction of different images for whatever reason, for example because of a 3D-Fast Fourier Transformation or because raw-data flows in so rapidly, while the reconstruction of a single image is very computationally intensive and thus different images may be reconstructed in parallel then.

A complicated reconstruction task may also comprise an MRI "parallel imaging" which is an acquisition technique to generate an image by reducing the number of acquired k-space lines (by the use of multiple receive channels). Image reconstruction for this task is quite compute intensive, therefor it is also advantageously divided in sub-tasks and distributed to different computing sources.

The sequence of consecutive sub-tasks is referred to the order in which the sub-tasks are processed, if they are all sequentially performed, only by the local control device. However, it is intended to distribute at least one sub-task to a remote computing source such that some sub-tasks are possibly processed in parallel.

Optionally, the local control device can comprise a first communication interface for transmitting the control data to a scan unit of the medical imaging system and for receiving the raw data from the scan unit.

Further, the local control device comprises an execution unit for executing the computing task. The execution of the computing task comprises a generation of computing results related to the generation of image data, in particular related to the generation of control data and/or to the processing of received raw data.

The local control device also comprises a selection unit for selecting a sub-task of the consecutive sub-tasks for external execution. As later described, such a sub-task can be selected based on the order of the reception of input data necessary for execution of the selected sub-tasks or based on the time consumption of the selected sub-tasks. Preferably, a sub-task with high time consumption is selected for external execution and/or a sub-task which is positioned on the back positions of a sequence of sub-tasks is selected for external execution.

Further, the local control device comprises an outsourcing unit for outsourcing the selected sub-task to a remote computing source for remotely generating a partial computing result and for receiving the generated partial computing result. A remote computing source may be any computing source outside of the medical imaging system, which comprises the local control device. For outsourcing the sub-task, the outsourcing unit preferably comprises a second communication interface for communication with a remote computing source.

The selection unit and/or the outsourcing unit may be encompassed by the measurement control unit, which may also execute the scheduling tasks of selecting and/or outsourcing the sub-tasks.

It has to be mentioned that the remote computing source may comprise a set of variable computing resources which set is not fix, but can and should adapt dynamically to the availability and thus scales dynamically and can even be extended to further computing resources if required e.g. on pay-peruse-basis in a cloud computing system. Preferably, the local control device for a medical imaging system itself offers its computing capabilities to other medical imaging systems if it is idle itself.

Hence, the local control device is arranged to execute at least one computing task, which is divided into a sequence of consecutive sub-tasks, and to transmit at least one sub-task to at least one remote computing source preferably through the second communication interface.

The local control device is arranged to receive computing results carried out by occasion in the at least one remote computing source preferably through the second communication interface and to use the received computing results for execution the computing task based on the received generated partial computing result. However, if the computing results of the remote computing source are not available due to any problems of data transmission or data procession or due to any other obstacle for an availability of the remotely computed results, the computing task is completed by the local control device as substitute. That is meant by the expression that the local control device performs the complete computing task by default.

Advantageously, the local control device is always self-contained and always able to execute the overall computing task on its own. It uses available free computing sources dynamically that are anyhow available or that are intentionally provided to speed-up computations. Further, computing resources of medical imaging systems which are usually only used when an imaging process is running, can be exploited over the whole time if they are accessed by remote medical imaging systems. Advantageously, time critical tasks in medical imaging, for example the execution of an image reconstruction, are accelerated using the remote computing source if available such that the results, in particular images, are available to an operator of the medical imaging system shortly after executing the measurement itself. Short time of execution of computing tasks, in particular tasks for executing a reconstruction, is crucial, especially in emergency circumstances where patient's health is at risk.

The medical imaging system, preferably an MR imaging system, according to the invention comprises a scan unit for acquiring raw data from an object to be examined and the local control device according to the invention. If the medical imaging system comprises an MR imaging system, the local control device may include a measurement control unit and an execution unit, in particular a reconstruction unit, for reconstructing image data based on acquired raw data. The medical imaging system shares the advantages of the local control device according to the invention.

The medical imaging systems may also include more than one medical imaging system, for example an ensemble of at least two medical imaging systems. In that case, at least one of the at least two medical imaging systems comprises a local control device also including an execution unit used as the remote computing source for remotely generating a partial computing result of a selected sub-task received from another medical imaging system. Of course, also more than one, for example all of the at least two medical imaging systems may use their local control device and in particular their execution unit as a remote computing source for remotely generating a partial computing result of a selected sub-task of another remotely located medical imaging system.

Preferably, the medical imaging system includes an ensemble of at least two medical imaging systems, wherein each of the at least two medical imaging systems comprises the local control device according to the invention and an execution unit also used as a remote computing source for remotely generating a partial computing result of the selected sub-task of another medical imaging system. Advantageously, the different medical imaging systems are enabled to exchange selected sub-tasks dependent on current free calculating capacities such that a medical imaging process of all these medical imaging systems of the ensemble is accelerated as much as possible. In case of a network failure, the system falls back to processing a complete task by one self-contained medical imaging system.

The system-cluster according to the invention comprises at least one medical imaging system according to the invention and at least one additional remote system including the remote computing source for remotely generating a partial computing result of the selected sub-task. The remote system includes technical means for performing tasks or sub-tasks outside of the at least one medical imaging system.

Preferably, the additional remote system comprises at least one of the following types of remote systems:
- a medical imaging system,
- a compute-only source.

As described above, a system-cluster including an ensemble of more than one medical imaging system enables to exchange selected sub-tasks dependent on current free calculating capacities such that a medical imaging process of all these medical imaging systems of the ensemble is accelerated as much as possible. In case of a network failure, the system-cluster falls back to processing a complete task by one self-contained medical imaging system.

A compute-only source does not include a complete medical imaging system, quite the contrary, it includes only a computer for calculating results of tasks or sub-tasks received from a medical imaging system or another compute-only source. Advantageously, the at least one compute-only source enables to further speed up the medical imaging process. For example, the system-cluster may include a dedicated computing cluster or one or more highly-performant computing machines with a single task of offering free computing resources that may be used by a medical imaging system which is still self-contained and can operate the complete task on its own in case of network issues. A big advantage of the system-cluster is that the additional computing power, i. e. the additional compute-only source, can easily be extended or renewed with a significantly higher frequency than the medical imaging system itself to benefit from the very short innovation cycles in the computing industry. Such a compute-only source can also be provided by a cloud computing system with of course higher latencies, but for some use cases this may also be beneficial, for example, if a very high amount of computation work is required only in rare cases.

The method for controlling a medical imaging system comprises the steps of dividing a computing task into a sequence of consecutive sub-tasks, wherein the computing task is related to a generation of image data based on measurement data. Further, the method according to the invention comprises the step of executing the computing task. For executing the task, a sub-task of the consecutive sub-tasks is selected for external execution. Then, the selected sub-task is outsourced to a remote computing source for remotely generating a partial computing result. The generated partial computing result is received and the computing task is executed based on the received generated partial computing result, in particular if there are further computing tasks depending on that result i.e. the step of executing the computing task comprises the step of executing the computing task based on the received generated partial computing result. The method for controlling a medical imaging system shares the advantages of the local control device according to the invention.

Some units or modules of the local control device mentioned above can be completely or partially realized as software modules running on a processor of a respective computing system, e.g. on a local control device for a medical imaging system, in particular a magnetic resonance imaging system or a CT-system. A realization largely in the form of software modules can have the advantage that applications already installed on an existing computing system can be updated, with relatively little effort, to install and run these units of the present application. The object of the invention is also achieved by a computer program product with a computer program that is directly loadable into the memory of a computing system, and which comprises program units to perform the steps of the inventive method, at least those steps that could be executed by a computer, especially the steps of dividing a computing task into a sequence of consecutive sub-tasks, executing the computing task, selecting a sub-task of the consecutive sub-tasks for external execution, outsourcing the selected sub-task to a remote computing source for remotely generating a partial computing result, receiving the generated partial computing result and executing the computing task based on the received generated partial computing result, when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

The dependent claims and the following description each contain particularly advantageous embodiments and developments of the invention. In particular, the claims of one claim category can also be further developed analogously to the dependent claims of another claim category. In addition, within the scope of the invention, the various features of different exemplary embodiments and claims can also be combined to form new exemplary embodiments.

In a preferred variant of the local control device according to the invention, the control task includes at least one of the following types of tasks:
- controlling a scan unit of the medical imaging system,
- reconstructing image data based on raw data acquired by a scan unit of the medical imaging system.

Advantageously, the generation of control data or a sub-task of a reconstruction task can be relocated to different computing resources for accelerating a control process or reconstruction process.

As later described in detail, also additional steps of a medical image generating process, in particular a filtering step or a post-processing of generated image data, are preferably carried out distributed, i.e. preferably in parts by the remote computing source or alternatively, in particular by default, by the local control device.

In a preferred variant of the local control device according to the invention, the computing task is executed based on a set of input data and the measurement control unit is arranged to divide the set of input data into sub-sets and to assign each of the sub-sets of input data to a different sub-task. The input data comprise data to be processed by the computing task, for example control data or raw data or image data. The separated sub-sets include the data to be processed by the related sub-tasks. Advantageously, by assigning the related input data to each of the sub-tasks, each of the sub-tasks can be processed independently from each other.

In a particular variant of the local control device according to the invention, the task comprises a reconstruction task for parallel imaging based on sub-sets of parallel acquired raw data and each of the sub-tasks comprises a reconstruction of a partial image based on a different sub-set.

Preferably, the execution unit is arranged to locally execute a sub-task, if an outsourcing of this sub-task is not appropriate according to a predefined criterion. A predefined criterion may comprise that the local execution is more efficient, preferably with respect to aspects of time consumption or existence of free computing resources.

In other words, the outsourced sub-task is locally executed by the execution unit of the local control device by default. "By default" means that the outsourced sub-task is executed by the local execution unit based on the above-mentioned criterion. The above-mentioned criterion preferably includes the decision of a locally execution of the sub-task in case no result is received from the remote computing unit processing the outsourced sub-task or no result is received under a predefined condition. Advantageously, the local control unit is self-contained and does not necessarily need the support of any remote computing unit.

In a variant of the local control device according to the invention, the execution unit is arranged to detect, as a special type of a predefined criterion or predefined condition, the current status or expected status of one or more consecutive sub-tasks, in particular of one or more completed consecutive sub-tasks or one or more consecutive sub-tasks expected to completed. The consecutive sub-tasks are preferably sub-tasks preceding the outsourced sub-tasks. The status of one or more of these consecutive sub-tasks is detected to determine based on the detected status if the partial computing result generated by the remote computing source is received by the local control device on time or will be received on time. Hence, the correct timing of the reception of the partial computing result depends on the status of completion of the consecutive sub-tasks, preferably the sub-task or sub-tasks preceding the outsourced sub-task.

Further, the criterion may also include the condition that the result of the outsourced sub-task is needed for the possibly existing sub-tasks being subsequent to the outsourced sub-task. Preferably, at the time point when the execution of the sub-tasks preceding the outsourced sub-task is finished or alternatively at the time point when the execution of the sub-tasks being subsequent to the outsourced sub-task is planned to be started, the result of the outsourced sub-task has to be received according to the predefined criterion.

Hence, it is detected if it is necessary to start the related sub-task by the local control device by default, i.e. as a substitute for the remotely executed sub-task. Further, the local control device is arranged to execute the computing task based on the received partial computing result if the partial computing result is received on time, or alternatively to execute the selected sub-task itself if the partial computing result is not received on time. Advantageously, the computing task can be completed in any case without any delay compared to a completely local execution of the computing task.

In another variant of the local control device according to the invention, the sequence of sub-tasks to be processed is implemented as a queue of consecutive sub-tasks. The queue determines an unambiguous order of processing the consecutive sub-tasks, particularly in case the sub-tasks are performed by the local control device. Advantageously, an order for processing the different sub-tasks is generated. For example, the order can be determined based on available input data or based on the consumption of time the different sub-tasks need.

In a preferred variant of the local control device according to the invention, the local control device includes a modularized execution unit, preferably a modularized reconstruction unit.

The modularized execution unit comprises a plurality of sub-units, including computing sources for performing a sub-task, preferably reconstruction sub-tasks. For example, the total imaging generation process includes inter alia a reconstruction task, wherein preferably a complete database of input data for that special reconstruction task is already available. As previously discussed, such a reconstruction task may include the parallel reconstruction of a predetermined number of sub-images based on raw data acquired in parallel. These raw data represent the complete database for the different sub-tasks of the reconstruction task.

Some of the different sub-units perform different computing sub-tasks. Hence, a computing task is split into several independent computing sub-tasks, wherein some of the different independent computing sub-tasks are performed by a different computing source, i.e. a different sub-unit. After computation, the results of the different independent computing sub-tasks are combined altogether to a combined final overall single result.

The sub-units may be implemented by a modularized hardware and/or software structure for executing the different sub-tasks. Preferably the execution unit includes individual computers. However, the sub-units can also be implemented by a single computer, wherein each function of the sub-units is executed by the single computer, but each function is still logically separated. Hence, the execution unit may include a single computer which comprises a modularized software structure for individually executing the different sub-tasks.

Hence, the principle of the modularized execution unit is to split up a monolithic task, preferably a reconstruction task, into certain chunks of independent computing sub-tasks that can be flexibly either executed on a local machine, i.e. the execution unit, or distributed to other places, for example remote computing sources, in particular remote medical imaging systems. The generated sub-tasks are already supplied with a necessary data basis of input data such that they can be transferred together with the assigned data basis of input data to a logically separated or physically separated computing source.

As mentioned above, the software structure used for the implementation of the distributed structure preferably comprises a queue, wherein the computing task is divided into the above-mentioned sub-tasks and these sub-tasks are stacked up and then distributed to different computing sources. It has to be mentioned that the queue only includes these selected sub-tasks ready to be processed at the point of time of selection. Preferably, the individual results of the selected sub-tasks are collected and put together to the final overall result in a reverse manner. The division of the computing task into sub-tasks is preferably organized by the measurement control unit.

The overall result, may comprise either a simple collection of individual results, in particular images, for a single measurement or something that needs to be at least partly computationally combined or further processed. Advantageously, the modularized structure of the possibly spatially distributed hardware for executing the computing task is mapped on the software and/or hardware structure of the execution unit of the local control device according to the invention such that a computing task is enabled to be processed in a modularized manner in each case, i.e. locally for default or by outsourcing at least one sub-task to a remote computing source.

In a further variant of the local control device according to the invention, the remote computing source includes a remote local control unit of a different medical imaging system. Advantageously, computing resources of medical imaging systems, which are usually only used when an imaging process is running, can be exploited over the whole time, if they are accessed by remote medical imaging systems.

Preferably, in a variant of the local control device according to the invention, the remote computing source includes a cluster comprising a plurality of local control units for different medical imaging systems. Advantageously, every medical imaging system that has an idle computing power can be used to execute sub-tasks, thus they form a kind of virtual cluster, distributed and dynamically available. Further, the local control device according to the invention itself may be arranged to offer its computing power to other remote medical imaging systems if it is idle itself.

In a preferred variant of the local control device according to the invention, the remote computing source is located in a cloud computing system. Advantageously, the remote computing source can also be provided as virtual resource in a cloud computing system without an actual existence of an additional remote medical imaging system.

Preferably, the medical imaging system according to the invention comprises at least one of:
- a magnetic resonance imaging system,
- an X-ray imaging system, preferably a CT-imaging system,
- an ultrasound imaging system.

A magnetic resonance imaging method comprises, additionally to the acquisition of raw data, a plurality of processing steps. These processing steps preferably include processing steps for preparing the raw data for reconstruction of image data, the reconstruction of image data itself and further steps for generating image data, for example the application of additional reconstruction algorithms, the combination of partial images to a combined image and post-processing steps for corrections of movements. Further, the steps for generating image data can comprise steps for generating an evaluation of time series, steps for detection of anatomical structures, steps for performing a functional magnetic resonance imaging, steps for performing a diffusion imaging, steps for performing a perfusion imaging and steps for performing filtering methods. All these steps can be divided in sub-steps, i.e. sub-tasks and can be performed in a distributed manner using a remote computing source.

Preferably, the reconstruction is divided into a plurality of sub-tasks, which are transmitted to remote computing resources. Further, also the following steps like application of additional reconstruction algorithms, post-processing steps for corrections of movements, evaluation of time series, detection of anatomical structures, functional magnetic resonance imaging, diffusion imaging, perfusion imaging and filtering methods, can be divided into sub-tasks and outsourced to different remote computing sources.

Input data are typically either pre-processed raw-data or reconstructed images (the latter especially for post-processing), but this is strongly dependent on the concrete algorithm or even variant. Intermediate points could make sense, but in practice, these two are the most relevant ones. For example, both filtering or motion correction can be done either on early stages in the image recon pipeline or on the reconstructed images, depending on the concrete algorithm. In practice, we have to identify a concrete point in the reconstruction pipeline at which it makes sense for the individual concrete use case from which on the external computation would make sense. For example, in case of alternative reconstructions on the basis of the same raw data this would be at a very early stage of the pipeline (directly after system-dependent pre-processing/normalization of the raw data), even if the original reconstruction offloaded only e.g. post-processing tasks (e.g. because the medical images, in particular MR images, are acquired sequentially over time).

A CT-imaging method comprises the steps of receiving raw data from a scan unit, a preparation step for generating corrected and re-arranged raw data. The actual CT-reconstruction step for generating image data based on the prepared raw data and a filtering step. In CT-imaging, a plurality of different sets of image data can be generated based on the same set of raw data using different sets of parameters for reconstruction. All these reconstructions can be implemented as sub-tasks performed on different remote computing sources. Further, a filtering step is carried out on the generated image data. Also the filtering step can be carried out by the different remote computing sources or alternatively by a local control device.

The invention is explained below with reference to the figures enclosed once again. The same components are provided with identical reference numbers in the various figures. The figures are usually not to scale.
FIG 1 shows an MR imaging system according to prior art,
FIG 2 shows an MR imaging system according to an embodiment of the invention,
FIG 3 shows an ensemble of MR imaging systems according to an embodiment of the invention,
FIG 4 shows an arrangement including an MR imaging system according to the invention,
FIG 5 shows a flow chart diagram illustrating a method for controlling a medical imaging system according to the invention,
FIG 6 shows a schematic view on a local control device according to an embodiment of the invention,
FIG 7 shows steps 5.II to 5.IX illustrated in FIG 5, schematically represented for an even more detailed understanding,
FIG 8 illustrates a plan for performing a reconstruction task,
FIG 9 shows a schematic view on the workflow of the reconstruction,
FIG 10 shows a roughly schematic representation of a magnetic resonance tomography system,
FIG 11 shows a flow chart diagram illustrating an embodiment of the method for controlling a medical imaging system applied to a parallel MR-imaging method,
FIG 12 shows a flow chart diagram illustrating an embodiment of the method for controlling a medical imaging system applied to a computer tomography method.

In FIG 1, a schematic view on two conventional medical imaging systems, a first conventional medical imaging system 10, in particular an MR system, and a second conventional medical imaging system 10', in particular an MR system, with a scan unit 3 and an associated control device 1, 1' also named measurement and reconstruction system (abbreviated by the acronym MARS) are shown. The first conventional medical imaging system 10 comprises a uniform control device 1 and the second conventional medical imaging system 10' comprises a modularized control device 1' including a first unit 4, also named measurement control unit 4 (abbreviated by the acronym MC), and a second unit 5, also named reconstruction unit 5 (abbreviated by RECON). Both types of conventional medical imaging systems 10, 10' include a monolithic computing model for image reconstruction, i.e. they run the whole computation like the image reconstruction as a single complex computing task. The modularized control device 1' of the second conventional medical imaging system 10' can be implemented as a logical separation within one single control device 1' or as two real separate entities, i.e. two separate computing units 4, 5. Hence, particularly, the reconstruction tasks in the second conventional medical imaging system 10' are performed by a monolithic reconstruction unit 5.

FIG 2 shows a schematic view on a medical imaging system 20 according to an embodiment of the invention. The medical imaging system 20, in particular a magnetic resonance imaging system, comprises a scan unit 3 and a local control device 21. Similar to the second variant 1' of a conventional control device for a medical imaging system in FIG 1, the local control device 21 in FIG 2 includes two separate units 4, 24, i.e. a measurement control unit 4 and a modularized reconstruction unit 24, also named local control unit. The modularized reconstruction unit 24 comprises a plurality of sub-units REC₁, ..., RECₘ, i.e. computing sources, for performing reconstruction sub-tasks, also named RECON-machines (m is a natural number). For example, the total imaging generation process includes inter alia a reconstruction task T, wherein preferably a complete database of input data for that special reconstruction task T is already available. As previously discussed, such a reconstruction task may include the parallel reconstruction of a predetermined number of sub-images based on raw data RD₁, ..., RDₙ acquired in parallel. These raw data represent the complete data base for the different sub-tasks T₁, ..., Tₙ of the reconstruction task T.

Some of the different sub-units REC₁, ..., RECₘ (with m <= n) perform different computation tasks T₁, ..., Tₙ. Hence, a reconstruction task T is split into several independent computation tasks T₁, ..., Tₙ, wherein some of the different independent computation tasks T₁, ..., Tₙ are performed by a different computing source, i.e. a different sub-unit REC₁, ..., RECₘ. After computation, the results of the different independent computation sub-tasks T₁, ..., Tₙ are combined altogether to a combined final overall single result.

The sub-units, also named RECON-machines REC₁, ..., RECₘ, can include individual computers, but they can also be implemented by a single machine which comprises several integrated entities or even a single MARS-computer, wherein each function of the different RECON-machines REC₁, ..., RECₘ is integrated but still logically separated.

Hence, the principle of the modularized reconstruction unit 24 in FIG 2 is to split up a monolithic reconstruction task T into certain chunks of independent computation sub-tasks T₁,..., Tₙ that can be flexibly either executed on a local machine, i.e. the reconstruction unit 24, or distributed to other places, for example remote computing sources or remote medical imaging systems. It has to be mentioned that the whole reconstruction process may include much more sub-tasks than the mentioned sub-tasks T₁,..., Tₙ. However, the generated sub-tasks T₁,..., Tₙ are already supplied with a necessary data basis of input data such that they can be transferred together with the assigned data basis of input data to a logically separated or physically separated computing source, in particular a separate RECON-machine REC₁, ..., RECₘ.

The software structure used for the implementation of the distributed structure is a queue Q, wherein the reconstruction is divided into the above-mentioned sub-tasks T₁,..., Tₙ, also named chunks and these chunks are stacked up and then distributed to different computing sources. It has to be mentioned that the queue Q only includes these selected sub-tasks T₁,..., Tₙ ready to be processed at the point of time of selection. Indeed, the individual results of the selected sub-tasks T₁,..., Tₙ are collected and put together to the final overall result in a reverse manner. The division of the tasks into sub-tasks T₁,..., Tₙ is organized by the measurement control unit 4. The queue Q is symbolized in the measurement control unit 4. The queue Q symbolizes that the different reconstruction sub-tasks T₁, ..., Tₙ are stacked up and then distributed. The individual results of the different sub-tasks T₁, ..., Tₙ are then collected and put together to the final overall result in a reverse manner by the local control device 21. The overall result (of an MR measurement) may be either a simple collection of individual results (e.g. images) for this single MR measurement or something that needs to be (at least partly) computationally combined or further processed. The first option is typically the most relevant in practice.

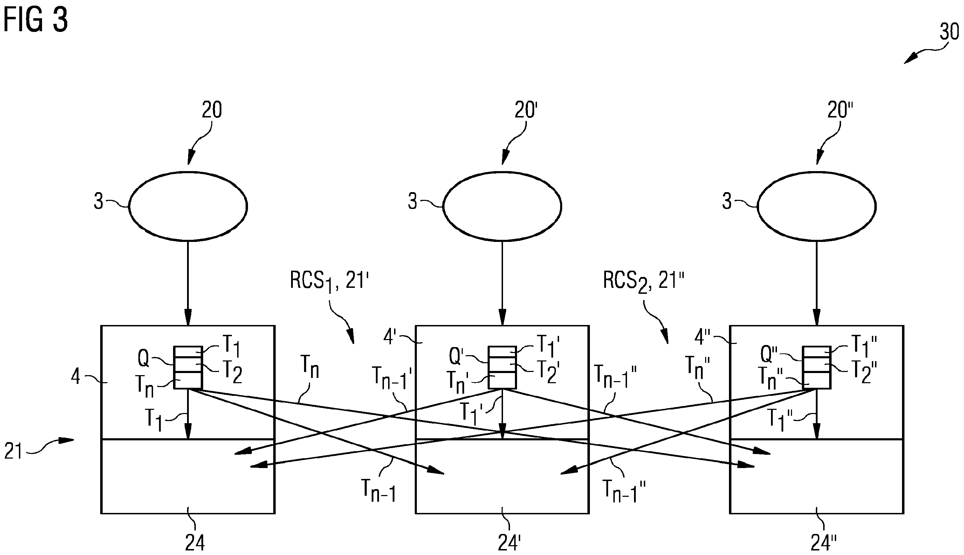

In FIG 3, an ensemble 30 of a plurality of possibly remotely located medical imaging systems 20, in particular three MR imaging systems 20, is visualized. Each of the MR imaging systems 20, 20', 20" is structured similar to the MR imaging system 20 shown in FIG 2. Hence, each of the three MR imaging systems 20, 20', 20" include a control device 21, 21', 21", wherein it depends on the perspective of the control device 21, 21', 21" if it is local or remote. Each control device 21, 21', 21" comprises a measurement control unit 4, 4', 4" and a modularized reconstruction unit 24, 24', 24". Although not shown, each of the modularized reconstruction units 24, 24', 24" are also divided into separate real or logic functional units, in particular RECON-machines REC₁, ..., RECₘ, in a cluster-style to process the chunks, i.e. the sub-tasks T₁, ..., Tₙ of a computing work, in particular a reconstruction task T.

However, in FIG 3 the different reconstruction sub-tasks T₁, ..., Tₙ are now additionally to the local control unit or reconstruction unit 24, at least partly, distributed to the other free computing resources, i.e. reconstruction units 24', 24" of the different MR imaging systems 20', 20" used as remote computing source RCS₁, RCS₂. Every MR imaging system 20', 20" that has an idle computing power may be used to execute such chunks of computing work, i.e. sub-tasks T₁, ..., Tₙ, thus the MR imaging systems 20, 20', 20" form a kind of virtual cluster, typically distributed and dynamically available.

Some of these computing sub-tasks T₁, ..., Tₙ are dynamically transmitted to the other MR imaging systems 20', 20" that are available and that have free computing resources available. In a worst case, the original MR imaging system 20 performs all of the computing sub-tasks T₁, ..., Tₙ alone. However, as soon as at least one or more computing sub-tasks T₁, ..., Tₙ are successfully executed by other supporting MR imaging systems 20', 20", the overall image reconstruction time is speed up accordingly. Vice versa, the other MR imaging systems 20', 20" also distribute their sub-tasks T₁',..., Tₙ', T₁",..., Tₙ" each structured in a software structure of a queue Q', Q" to remote imaging systems 20, 20" or remote imaging systems 20, 20' respectively, for execution.

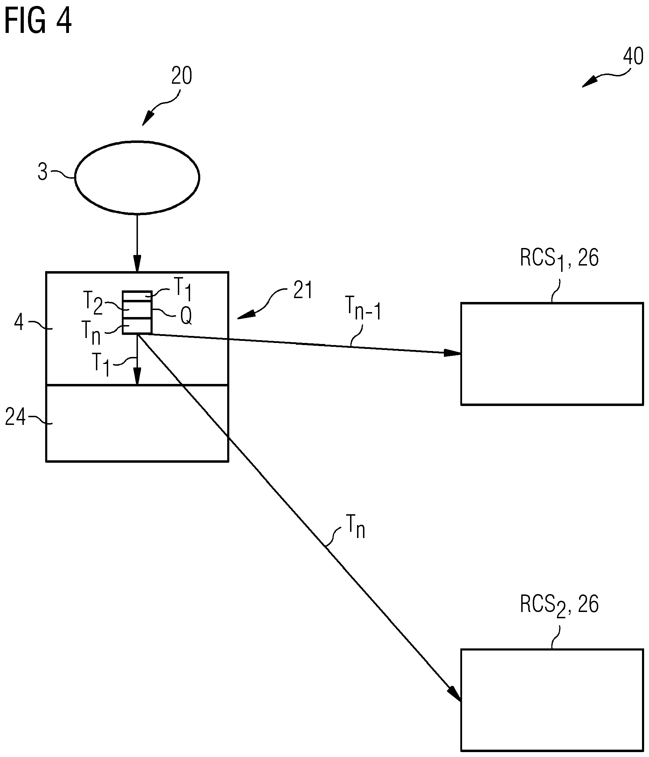

In FIG 4, a combination 40, i.e. a dedicated computing cluster of an MR imaging system 20 according to the invention and additional remotely located dedicated compute-only units 26 used as remote computing resources RCS₁, RCS₂, are illustrated. The distribution of computing power amongst each other is enriched by dedicated compute-only units 26 to further speed up computing time. Hence, a dedicated computing cluster includes a plurality of highly-performant computing machines, i.e. compute-only units 26 with a single task of offering free computing resources that may be used by the MR imaging system 20 is realized. As can be taken from FIG 4, the different sub-tasks Tₙ₋₁, Tₙ are transmitted to the compute-only units 26, wherein other sub-tasks T₁, T₂ remain in the MR imaging system 20 such that the MR imaging system 20 can start with these sub-tasks T₁, T₂, wherein in parallel the other sub-tasks Tₙ₋₁, Tₙ are processed in the compute-only units 26. As soon as these sub-tasks Tₙ₋₁, Tₙ are finished, the remote compute-only units 26 continue with other not yet processed sub-tasks Tₙ₋₂ and so forth, until all task are finished.

After finishing the local processed sub-tasks T₁, T₂, the local control device 21 checks if the results of the remotely processed sub-tasks Tₙ₋₁, Tₙ have been finished. Otherwise, the local reconstruction unit 24 of the MR imaging system processes these not yet finished sub-tasks Tₙ₋₁, Tₙ by default such that the reconstruction process is not interrupted or delayed.

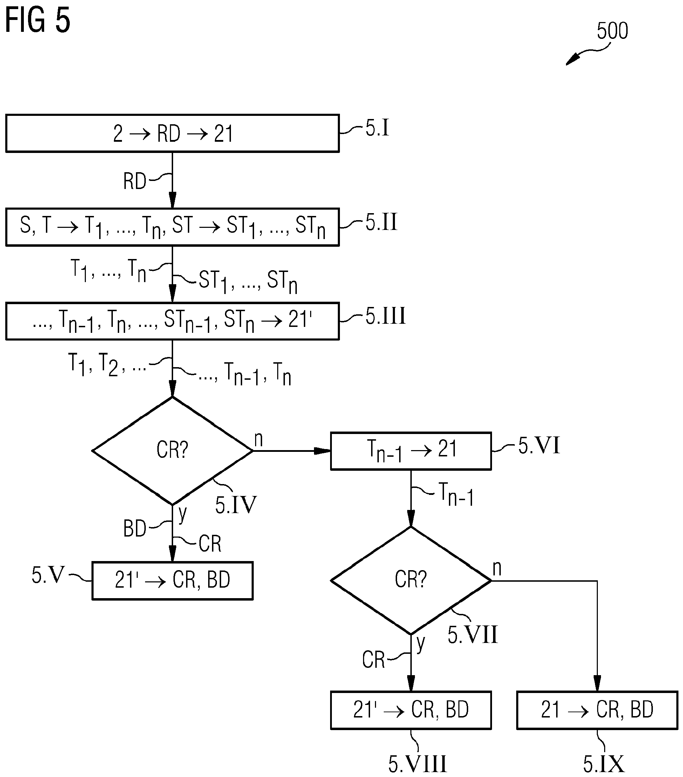

In FIG 5, a flow chart diagram 500 illustrating a method for controlling a medical imaging system, in particular a magnetic resonance imaging system 20 (as depicted in FIG 2 to 4), is shown.

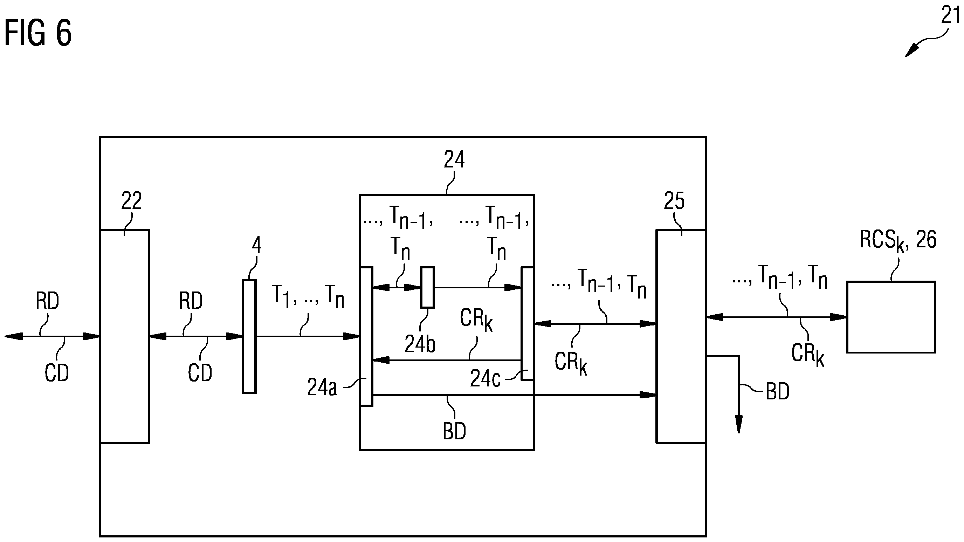

In step 5.1, a scan unit 2 of the magnetic resonance imaging system 20 is controlled by a local control device 21 (shown in FIG 6) through a control interface 22 (shown in FIG 6) for generating magnetic resonance signals, which are detected as raw data RD and are sent back to the local control device 21 through the control interface 22.

In step 5.II, a computing task T, in particular a predetermined reconstruction sequence task T for reconstructing images based on raw data acquired in parallel, which comprises a plurality of sub-tasks T₁, ..., Tₙ, in particular parallel reconstruction steps, is started for reconstructing image data BD. The sub-tasks T₁, ..., Tₙ are planned to be carried out based on a predetermined sequence S. The sequence S may be determined based on the time the sub-tasks T₁,..., Tₙ probably consume. Then, the sub-tasks with less time consumption are assigned to the local computing source and the more time consuming sub-tasks are assigned to the remote computing sources.

The sub-tasks ...,Tₙ₋₁, Tₙ later to be carried out in the sequence S are particularly appropriate for sourcing out to a local control device 21' (shown in FIG 3) of a remote magnetic resonance imaging system 20' (shown in FIG 3). Hence these sub-tasks ...,Tₙ₋₁, Tₙ are selected for transmitting these sub-tasks ...,Tₙ₋₁, Tₙ to the remote magnetic resonance imaging system 20'. In this way, the sub-tasks T₁, T₂,... which are on the front positions in the sequence S can be processed in the local control device 21 without any hesitation and the remote magnetic resonance imaging system 20' has more time to process the sub-tasks being on the rear positions of the sequence S.

The computing task T is executed based on a set ST of input data each related to a different sub-task T₁, ..., Tₙ. The set ST of input data, for example raw data, is divided into separate sub-sets ST₁, ..., STₙ to assign each of the separated sub-sets ST₁, ..., STₙ to the related sub-task T₁, ..., Tₙ.

In step 5.III, the selected sub-tasks ..., Tₙ₋₁, Tₙ are transmitted together with the related sub-sets ..., STₙ₋₁, STₙ to a remote control device 21' of the remote magnetic resonance imaging system 20' and the remaining, i.e. the sub-tasks T₁, T₂,... not selected for remote processing and the related sub-sets ST₁, ST₂ ..., are locally processed by the local control device 21.

In step 5.IV, the sequence S has been processed up to the selected and outsourced sub-tasks ..., Tₙ₋₁, Tₙ. At that point of time, it is checked if computing results CR, i.e. in particular image data BD, related to the selected and outsourced sub-tasks ..., Tₙ₋₁, Tₙ are received from the remote control device 21' of the remote magnetic resonance imaging system 20'. If the computing results CR, in particular image data BD, of the selected and outsourced sub-tasks ..., Tₙ₋₁, Tₙ are received from the remote control device 21' of the remote magnetic resonance imaging system 20', which is symbolized with "y" in FIG 5, then the computing results CR, i.e. image data BD, of the selected and outsourced sub-tasks ..., Tₙ₋₁, Tₙ are combined with other computing results CR from the locally processed sub-tasks T₁, T₂,... in step 5.V.

If not all of the computing results CR are received from the remote magnetic resonance imaging system 20', which is symbolized in FIG 5 with "n", then the sub-task Tₙ₋₁ assigned to the not received result CR is automatically started on the local control device 21 in step 5.VI.

In step 5.VII, during locally processing the selected sub-task Tₙ₋₁, it is checked if the computing results CR of the selected sub-task Tₙ₋₁ are received before completing the selected sub-task Tₙ₋₁ on the local control device 21. If the computing results CR, in particular image data BD, are received from the remote control device 21' before completing the selected sub-task Tₙ₋₁ on the local control device 21, which is symbolized in FIG 5 with "y", then, the local procession of the selected sub-task Tₙ₋₁ is stopped and the result CR assigned to the selected sub-task Tₙ₋₁ is combined with the results CR of the other sub-tasks in step 5.VIII.

If the computing results CR are not received from the remote control device 21' before completing the selected sub-task Tₙ₋₁ on the local control device 21, which is symbolized in FIG 5 with "n", then the sub-task Tₙ₋₁ is finished in the local control device 21 and the assigned computing results CR, in particular image data BD, are received from the local control device 21 in step 5.IX. Further, the computing results CR, in particular image data BD, of the different sub-tasks T₁, ..., Tₙ and resources are combined for generating a final result, in particular a combined image, of the reconstruction.

In FIG 6, a schematic view on a local control device 21 for a medical imaging system 20 (shown in FIG 3, 4) is illustrated. The local control device 21 comprises a first communication interface 22 for transmitting control data CD to a scan unit 3 (shown in FIG 3, 4) of the medical imaging system 20 and for receiving raw data RD from the scan unit 3. Further, the local control device 21 includes a local control unit 24 for performing a computing task T related to the control data CD and/or to the received raw data RD. The execution of the computing task T comprises a generation of computing results CR related to the control data CD and/or to the received raw data RD. The local control device 21 also includes a second communication interface 25 for communication with a remote computing source 26 (also shown in FIG 4). The local control device 21 is arranged to execute a computing task T which is divided into a sequence S of consecutive sub-tasks T₁, ..., Tₙ and to transmit a sub-task ..., Tₙ₋₁, Tₙ to the remote computing source RCSₖ, for example a compute only unit 26, (only one single remote computing source RCSₖ is depicted, however, a plurality of remote computing sources can be used for processing the different sub-tasks ..., Tₙ₋₁, Tₙ in parallel) through the second communication interface 25, to receive computing results CRₖ carried out by occasion in the remote computing source RCSₖ and to use the received computing results CRₖ for execution of the computing task T.

In detail, the local control device 21 comprises a measurement control unit 4 for dividing a computing task T into a sequence S of consecutive sub-tasks T₁, ..., Tₙ. Further, the local control device 21 comprises a local control unit 24.

In detail, the local control unit 24 comprises an execution unit 24a for executing the computing task T divided into consecutive sub-tasks T₁, ..., Tₙ. The local control unit 24, in that embodiment a reconstruction unit 24, also includes a selection unit 24b for selecting a sub-task ..., Tₙ₋₁, Tₙ of the consecutive sub-tasks T₁, ..., Tₙ for external execution and an outsourcing unit 24c for outsourcing the selected sub-task ..., Tₙ₋₁, Tₙ to a remote computing source RCSₖ, for example a compute-only unit 26, for remotely generating a partial computing result CRₖ and for receiving the generated partial computing result CRₖ. The execution unit 24a is arranged to execute the computing task T based on the received generated partial computing result CRₖ and for generating and emitting the final result, in particular the final image data BD through the second communication interface 25.

Selecting and outsourcing of the sub-tasks can in principle be done either in the control unit 4 or the reconstruction unit 24.

For the first variant speaks that the control unit 4 is close to "systems knowledge" and one can better control the overall behavior, especially since the process is in or at least very close to the real-time activities of the machine, i.e. the scan unit 3. Additionally, the reconstruction unit 24 does not need to be aware of this task of outsourcing and thus can simply concentrate on the task of computing and thus all computing units (including the remote computing source 26) can then be implemented very similar (this makes the scaling over different hardware much easier).

But also the other approach, to implement the scheduling of sub-tasks in the reconstruction unit 24 may have advantages: The control-unit 4 is not affected by its main task (including real-time tasks related to raw data acquisition and preprocessing, which barely make sense to outsource). The reconstruction unit 24 then automatically gets the more complex or comprehensive computing tasks related to image reconstruction only and can (even better, since reconstruction is its core knowledge) decide on its own, how to split up and distribute computation.

The components of the local control device 21 can be implemented predominantly or completely in the form of software elements on a suitable processor. In particular, the interfaces between these components can also be designed purely in terms of software. All that is required if there are access options to suitable storage areas in which the data can be stored temporarily and called up and updated at any time.

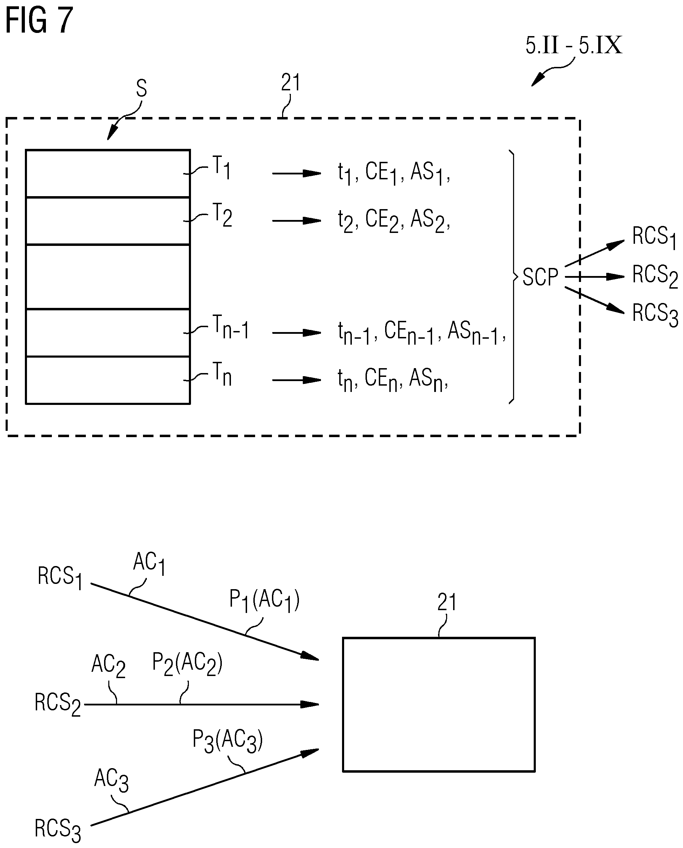

In FIG 7, steps 5.II to 5.IX illustrated in FIG 5 are schematically represented for an even more detailed understanding of the principles of the execution of the method for controlling a medical imaging system according to the invention.

In step 5.II, a computing task T related to a predetermined reconstruction sequence S which comprises a plurality of sub-tasks T₁, ..., Tₙ, in particular reconstruction steps, is planned. The sub-tasks T₁,..., Tₙ are planned to be carried out based on the predetermined sequence S. For planning the computing task T, a set SCP of characteristic parameters for each subtask T₁,..., Tₙ is calculated. To each of these individual sub-tasks T₁,..., Tₙ, a time interval t₁, ..., tₙ for the time requirement for performing each individual sub-task T₁, ..., Tₙ is assigned and an estimated computational effort CE₁, .., CEₙ and amount of storage AS₁, ..., ASₙ is calculated based on the type of the individual sub-task T₁, ..., Tₙ and the received amount of raw data RD. The computational effort CE₁, .., CEₙ can be calculated based on the complexity of an algorithm related to the respective sub-task T₁, ..., Tₙ and the amount of raw data RD. Hence, the set SCP of characteristic parameters comprises the following parameters:
time interval t: t₁, ..., tₙ,
computational effort CE: CE₁, .., CEₙ,
amount of storage AS: AS₁, ..., ASₙ.

The calculation of the set SCP of characteristic parameters can also be performed based on experimental or empiric data.

Further, a request is started and transmitted to all potential remote computing sources RCSₖ (k = 1..., m, in FIG 7: m = 3) (shown in FIG 3 and FIG 4), wherein the determined set SCP of characteristic parameters is transmitted to each of the remote computing sources RCSₖ.

After that, each of the requested remote computing sources RCSₖ replies by transmitting an answer including the free capacities for performing at least one or more sub-tasks T₁, ..., Tₙ.

Optionally, each remote computing source RCSₖ calculates itself based on the set SCP of characteristic parameters, which of the sub-tasks T₁, ..., Tₙ the respective remote computing source RCSₖ is able to perform in which amount of time, i.e. in which time interval t₁ₖ, ..., tₙₖ. Alternatively, each remote calculating source RCSₖ transmits a set representing its available capacity ACₖ, for example including its computation performance CPₖ, its available amount of storage ASₖ and its available calculation time atₖ and for example the price Pₖ(ACₖ) for the usage of the available capacity.

Based on the set SCP of characteristic parameters, a set of the received information related to available capacities ACₖ and a set of received assigned prices Pₖ(ACₖ), an optimization for example by generating a time function t_{F} =f (T, SCP, ACₖ) and a cost function c_{F} = f(Pₖ₍ACₖ₎), can be determined in the local control device 21.

Further, it has to be respected that an individual sub-task T₁, ..., Tₙ can be only started if the directly preceding sub-task T₁, ..., Tₙ has been finished, if there are data dependencies.

Taking into account that information, an optimal plan for performing the reconstruction task T is completed.

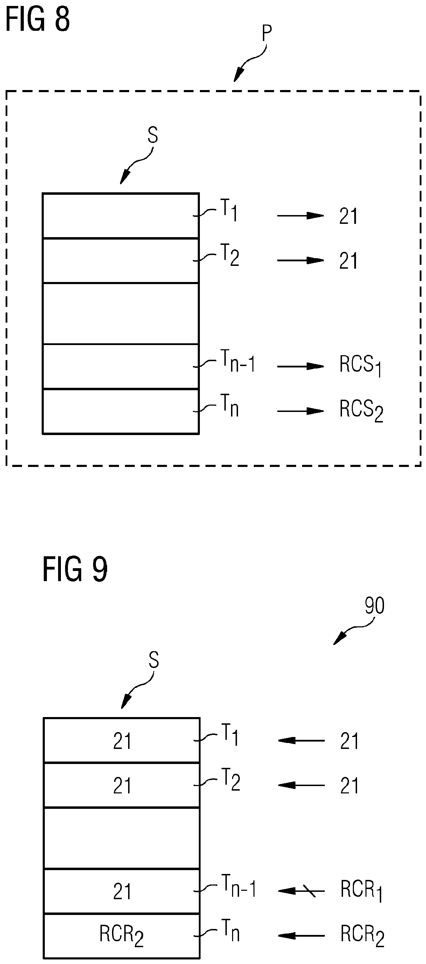

Such a plan P is shown in FIG 8. To each of the sub-tasks T₁, .., Tₙ, an individual calculating source is assigned. For example, the first and the second sub-task T₁, T₂ are performed by the local control device 21. The n-1-th sub-task Tₙ₋₁ is performed by a first remote calculating source RCS₁, and the n-th sub-task Tₙ is performed by a second remote calculating source RCS₂.

After that, a reconstruction T is started. In FIG 9, a schematic view 90 on the workflow of the reconstruction task T is illustrated. After finishing the n-2-th sub-task, the first remote calculating resource RCS₁ has not finished its calculation, for example due to a problem of transmitting data. Hence, the local control device 21 starts itself vicariously performing the n-1-th sub-task Tₙ₋₁ and generates results CRₙ₋₁ or distributes it to another remote computing device if e.g. that one is much more powerful than it is itself. At last, the n-th sub-task Tₙ is finished just in time by the second remote calculating source RCS₂, and the reconstruction task T can be completed based on the result of the n-th sub-task Tₙ finished by the second remote computing source RCS₂ and all computing results are combined. Consequently, the reconstruction task T is finished in reality in an optimum of time and optionally for an optimized amount of costs.

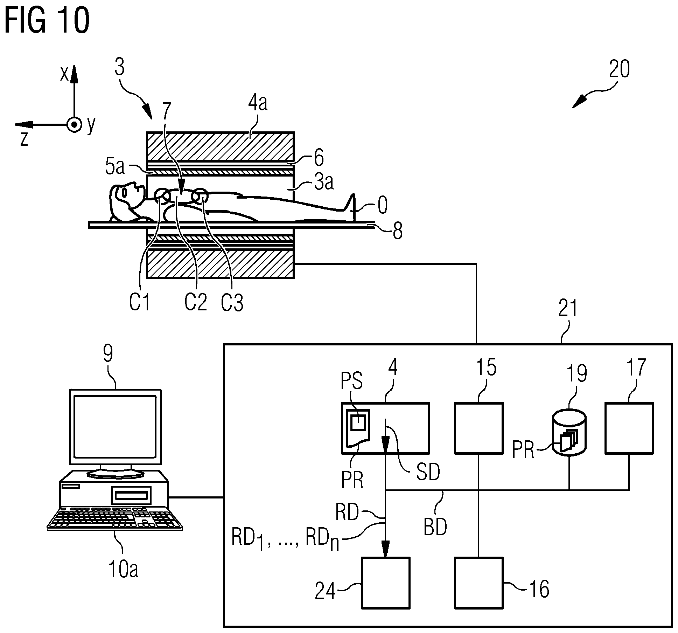

FIG 10 shows a roughly schematic representation of a magnetic resonance imaging system 20 according to the invention (hereinafter also referred to as "MR system" for short), including a local control device 21, which is designed to carry out a method for controlling an MR system according to the invention. On the one hand, the MR system includes the actual magnetic resonance scan unit 3 with an examination room 3a or patient tunnel, in which an examination object O, or here a patient or test person, is placed on a bed 8, in whose body the examination object, for example a specific organ located, can be retracted.

The magnetic resonance scan unit 3 is equipped in the usual way with a basic field magnet system 4a, a gradient system 6 and an HF transmission antenna system 5a and an HF reception antenna system 7. In the exemplary embodiment shown, the HF transmission antenna system 5a is a whole-body coil permanently installed in the magnetic resonance scan unit 3. The HF reception antenna system 7 comprises local coils with reception coils C1, C2, C3 to be arranged on the patient or subject O (in FIG 10 this is symbolized by three receiving coils C1, C2, C3 (usually there are several receiving coils). The receiving coils C1, C2, C3 are usually grouped in units with a common housing, which are often referred to as local coils. Several reception coils are available for parallel measurement.

The basic field magnet system 4a is designed here in the usual way so that there is a basic magnetic field in the longitudinal direction of the examination object O, in particular a patient, i.e. along the longitudinal axis of the magnetic resonance scan unit 3 running in the z-direction. The gradient system 6 includes gradient coils that can be controlled individually in the usual way, in order to be able to switch gradients in the x, y, or z direction independently of one another.

The MR system 20 also has a local control device 21 which is used to control the MR system 20. This central local control device 21 includes a sequence control unit or measurement control unit 4 for measurement sequence control. This is used to record the sequence of high-frequency pulses (HF pulses) and gradient pulses as a function of a selected pulse sequence PS or a sequence of multiple pulse sequences for recording multiple slices or volumes in a volume region of interest of the examination object O.

Such a pulse sequence PS can be specified and parameterized within a measurement or control protocol PR, for example. Different control protocols PR for different measurements or measurement sessions are usually stored in a memory 19 and can be selected by an operator (and changed if necessary) and then used to carry out the measurement. In the present case, a pulse sequence is selected in such a way that raw data is acquired in parallel with a plurality of receiving coils in the desired raster in a subsampled manner and for each coil, a different raw data set RD₁, ..., RDₙ is received.

To output the individual HF pulses of a pulse sequence PS, the local control device 21 has a high-frequency transmission device 15 that generates and amplifies the HF pulses and feeds them into the HF transmission antenna system 5a via a suitable interface (not shown in detail). The local control device 21 has a gradient system interface 16 for controlling the gradient coils of the gradient system 6 in order to switch the gradient pulses appropriately according to the predetermined pulse sequence. The measurement control unit 4 communicates in a suitable manner, e.g. by sending sequence control data SD, with the high-frequency transmission device 15 and the gradient system interface 16 for executing the pulse sequences. The local control device 21 has also a high-frequency receiving device 17 (which also communicates in a suitable manner with the measurement control unit 4). The receiving device 17 receives magnetic resonance signals within the readout window specified by the pulse sequence PS in a coordinated manner by means of the HF reception antenna system 7 and so on to acquire the raw data RD, including raw data sets RD₁, ..., RDₙ after demodulation and digitization.

A reconstruction unit 24 accepts the acquired raw data sets RD₁, ..., RDₙ at a raw data interface, also called a receiving unit, and reconstructs image data BD for the desired visual field from them. This reconstruction is also usually based on parameters that are specified in the respective measurement protocol.

In the present case, the reconstruction unit 24 is designed in such a way that it can work according to the method according to the invention, in particular for parallel reconstruction based on the different receiving coils C1, C2, C3.

The local control device 21 can be operated via a terminal with an input unit 10a and a display unit 9, via which the entire MR system 20 can therefore also be operated by an operator. MR images can also be displayed on the display unit 9, and measurements can be planned and started using the input unit 10a, possibly in combination with the display unit 9, and in particular control protocols PR with suitable pulse sequences PS can be selected as explained above and modified if necessary.

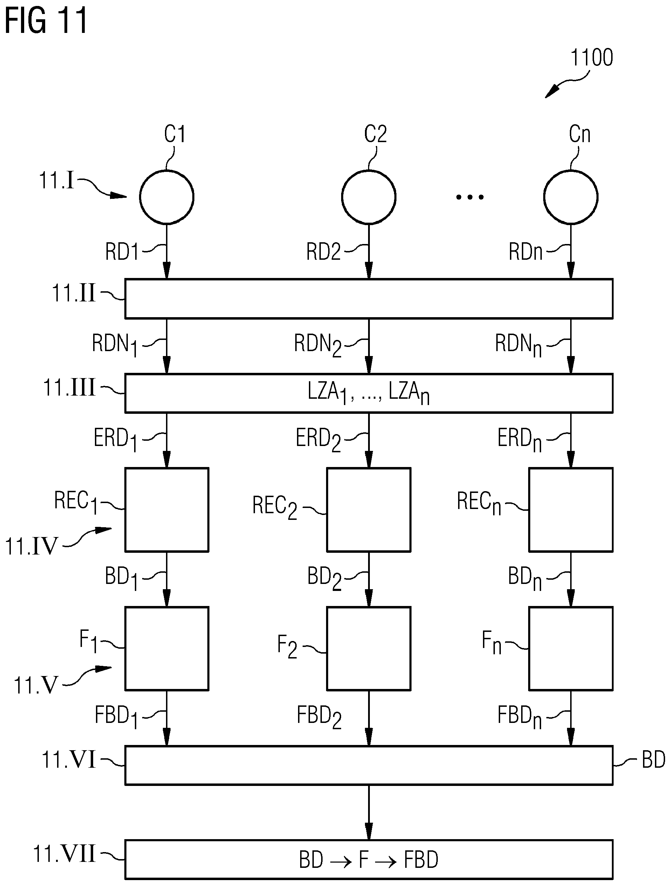

In FIG 11, a flow chart diagram 1100 illustrating an embodiment of the method according to the invention related to parallel MR imaging is shown.

First, in step 11.I, raw data sets RD₁, ..., RDₙ are acquired on different channels using a plurality of reception coils C1, C2, ..., Cn arranged on a patient. In step 11.11, the different raw data RD₁, ..., RDₙ are then normalized in the local control device 21.

After that, the normalized raw data RDN₁, ..., RDNₙ still comprise so-called blank lines or empty lines. Therefore, in step 11.III, sub-tasks LZA₁, ..., LZAₙ for adding empty lines are applied on the normalized raw data RDN₁, ..., RDNₙ such that complemented raw data ERD₁, ..., ERDₙ are generated. Also these sub-tasks LZA₁, ..., LZAₙ are usually locally performed due to their relatively low calculating amount.

Then, in step 11.IV, the complemented raw data ERD₁, ..., ERDₙ are assigned to different reconstruction sub-tasks REC₁, ..., RECₙ and some of these sub-tasks REC₁, ..., RECₙ are then transmitted to remote computing sources. Further, image data BD₁, ..., BDₙ are generated based on the complemented raw data ERD₁, ..., ERDₙ by the local control device 21 and also by the remote computing sources.

Further, in step 11.V, the generated image data BD₁, ..., BDₙ results are assigned to filtering sub-tasks F₁, ..., Fₙ and the filtering sub-tasks F₁, ..., Fₙ and the generated partial image data BD₁, ..., BDₙ are transmitted to remote computing resources for generating filtered image data FBD₁, ..., FBDₙ. After that, in step 11.VI, the filtered image data FBD₁, ..., FBDₙ are combined to a combined image BD by the local control device 21. In step 11.VII, an additional filter step F is applied to the combined image BD by the local control device 21.

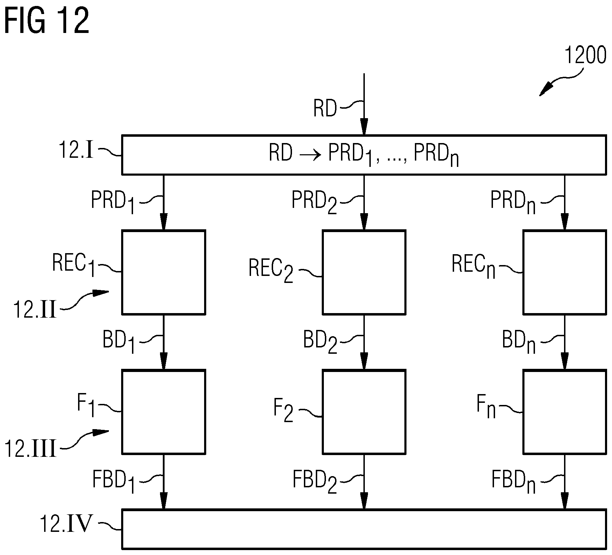

In FIG 12, a flow chart diagram 1200 illustrating an embodiment of the method according to the invention related to CT imaging is shown.

In step 12.1, raw data RD are received by a local control device from a scan unit of a CT-imaging system. Further, the raw data RD are locally prepared by correcting and rearranging the raw data RD such that prepared raw data PRD₁, ..., PRDₙ are generated.

In step 12.11, a reconstruction task is divided into a plurality of sub-tasks REC₁, ..., RECₙ assigned to different sets of prepared raw data PRD₁, ..., PRDₙ and these sub-tasks REC₁, ..., RECₙ are transmitted to remote computing sources. Further, the prepared raw data PRD₁, ..., PRDₙ are used for generating reconstructed image data BD₁, ..., BDₙ.

In step 12.III, the generated image data BD₁, ..., BDₙ are processed by remotely implemented filters F₁,..., Fₙ. The generated filtered image data FBD₁, ..., FBDₙ are then sent back to the local control device of the CT imaging system.

The above descriptions are merely preferred embodiments of the present disclosure, but not intended to limit the present disclosure, and any modifications, equivalent replacements, improvements, etc. made within the spirit and principle of the present disclosure should be included within the scope of protection of the present disclosure.

Further, the use of the undefined article "a" or "one" does not exclude that the referred features can also be present several times. Likewise, the term "unit" or "device" does not exclude that it consists of several components, which may also be spatially distributed.

## Claims

1. Local control device (21) for a medical imaging system (20), comprising:
- a measurement control unit (4) for dividing a computing task (T) into a sequence (S) of consecutive sub-tasks (T₁, ..., Tₙ), wherein the computing task (T) is related to a generation of image data (BD) based on measurement data,
- an execution unit (24a) for executing the computing task (T),
- a selection unit (24b) for selecting a sub-task (..., Tₙ₋₁, Tₙ) of the consecutive sub-tasks (T₁, ..., Tₙ) for external execution,
- an outsourcing unit (24c)
- for outsourcing the selected sub-task (..., Tₙ₋₁, Tₙ) to a remote computing source (RCSₖ, 21', 21", 26) for remotely generating a partial computing result (CRₖ) of the selected sub-task (..., Tₙ₋₁, Tₙ) and
- for receiving the generated partial computing result (CRₖ),
wherein the execution unit (24a) is arranged to execute the computing task (T) based on the received generated partial computing result (CRₖ).

2. Local control device according to claim 1, wherein the computing task (T) includes at least one of the following types of control tasks:
- controlling a scan unit (3) of the medical imaging system (20),
- reconstructing image data (BD) based on raw data (RD) acquired by a scan unit (3) of the medical imaging system (20) .

3. Local control device according to claim 1 or claim 2, wherein the computing task (T) is executed based on a set (ST) of input data and the measurement control unit (4) is arranged to divide the set (ST) of input data into sub-sets (ST₁, ..., STₙ) and to assign each of the sub-sets (ST₁, ..., STₙ) to a different sub-task (T₁, ..., Tₙ).

4. Local control device according to claim 3, wherein the task (T) comprises a reconstruction task for parallel image reconstruction based on sub-sets (ST₁, ..., STₙ) of parallel acquired raw data (RD) and each of the sub-tasks (T₁, ..., Tₙ) comprises a reconstruction of a partial image (BD₁, ..., BDₙ) based on a different sub-set (ST₁, ..., STₙ) .

5. Local control device according to any of the preceding claims, wherein the execution unit (24a) is arranged to locally execute a sub-task (..., Tₙ₋₁, Tₙ), if an outsourcing of this sub-task (..., Tₙ₋₁, Tₙ) is not appropriate according to a predefined criterion.

6. Local control device according to any of the preceding claims, wherein the execution unit (24a) is arranged
- to detect the status of a completed consecutive sub-task (T₁, ..., Tₙ) ,
- to determine based on the detected status if the partial computing result (CRₖ) generated by the remote computing source (RCSₖ, 21', 21", 26) is received by the local control device (21) or will be received on time, and
- to execute the computing task (T) based on the received partial computing result (CRₖ) if the partial computing result (CRₖ) is received on time, or alternatively
- to execute the selected sub-task (..., Tₙ₋₁, Tₙ) itself if the partial computing result (CRₖ) is not received on time.

7. Local control device according to any of the preceding claims, wherein the remote computing source (RCSₖ, 21', 21", 26) includes a remote local control device (21') of a remote medical imaging system (20').

8. Local control device according to any of the preceding claims, wherein the remote computing source (RCSₖ,, 21, 21', 26) includes a cluster comprising a plurality of remote local control devices (21', 21") for remote medical imaging systems (20', 20").

9. Local control device according to any of the preceding claims, wherein the remote computing source (RCSₖ, 21', 21', 26) is located in a cloud computing system.

10. Medical imaging system (20), comprising:
- a scan unit (3) for acquiring raw data (RD) from an examination object (O),
- a local control device (21) according to any of the preceding claims.

11. System-cluster (40), comprising:
- at least one medical imaging system (20) according to claim 10,
- at least one additional remote system (20', 20", RCS₁, RCS₂) including the remote computing source (RCSₖ, 21', 21", 26) for remotely generating a partial computing result (CRₖ) of the selected sub-task (..., Tₙ₋₁, Tₙ).

12. System-cluster (40) according to claim 11, wherein the additional remote system (20', 20", RCS₁, RCS₂) comprises at least one of the following types of remote systems:
- a medical imaging system (20', 20"),
- a compute-only source (RCS₁, RCS₂).

13. Method for controlling a medical imaging system (20), comprising the steps of:
- dividing a computing task (T) into a sequence (S) of consecutive sub-tasks (T₁, ..., Tₙ), wherein the computing task (T) is related to a generation of image data (BD),
- executing the computing task (T),
- selecting a sub-task (..., Tₙ₋₁, Tₙ) of the consecutive sub-tasks (T₁, ..., Tₙ) for external execution,
- outsourcing the selected sub-task (..., Tₙ₋₁, Tₙ) to a remote computing source (RCSₖ, 21a', 21a", 26) for remotely generating a partial computing result (CRₖ),
- receiving the generated partial computing result (CRₖ), wherein the step of executing the computing task (T) comprises the step of executing the computing task (T) based on the received generated partial computing result (CRₖ).

14. Computer program product with a computer program, which can be loaded directly into a memory device of a medical imaging system (20), with program sections to perform all the steps of the method according to claim 13, when the computer program is carried out in the medical imaging system (20).

15. Computer readable medium, on which program sections that can be read in and executed by a computer unit are stored in order to carry out all steps of the method according to claim 13, when the program sections are executed by the computer unit.
